# EUROPEAN PATENT APPLICATION

(11) **EP 4 219 511 A1**
(43) Date of publication of application: **02.08.2023**
(21) Application number: 23152200.4
(22) Date of filing: 18.01.2023
(51) Int. Cl.: C07F 5/02, C07D 201/00

(54) **INTERMEDIATE, PREPARING METHOD THEREOF, AND METHOD OF PREPARING DRUG**

(30) Priority: 27.01.2022 US 202263303978 P; 13.01.2023 TW 112101650
(71) Applicant: Heron Neutron Medical Corp., Hsinchu County 302 (TW)
(72) Inventor: HSIEH, Teng-San, 302 Zhubei City (TW); YU, Yu-Hou, 302 Zhubei City (TW); LIN, Tzung-Yi, 302 Zhubei City (TW)
(74) Representative: Berggren Oy

(57) **Abstract**

An intermediate is provided and has the structure shown in formula (1) as follows: 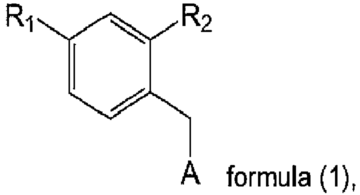 in which R₁ is -Cl, -Br, -I, -OSO₂CF₃, -B(OH)₂, or 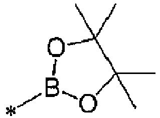 , R₂ is -F, -¹⁸F, -Cl, -Br, -I, -SnMe₃, -SnBu₃, -B(OH)₂, or 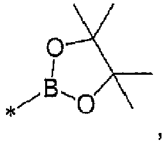 and A is a chiral auxiliary.

## Description

### BACKGROUND

### Field of Invention

The present disclosure relates to an intermediate, a preparing method thereof, and a method of preparing a drug from an intermediate.

### Description of Related Art

Malignant tumors as serious diseases seriously endanger human life and health. At present, a method of treating patients suffering from malignant tumors has been developed by radiotherapy. For example, the process of boron neutron capture therapy (BNCT) is as the following: injecting a boron-containing drug into the body of the patients, the boron-containing drug selectively accumulating in the tumor cells, and boron and neutron in the tumor cells undergoing nuclear fission after irradiating with a neutron beam to generate an alpha particle and a lithium-ion for precisely destroying the tumor cells. During the BNCT, the distribution position and concentration of the boron-containing drug in the patient can be confirmed by positron emission tomography (PET) to ensure that the normal cells are not damaged during the BNCT and that the treatment meets the expected effect.

In view of the importance of the boron-containing drug for treating malignant tumors, it is necessary to develop a novel and efficient method of preparing a boron-containing drug.

### SUMMARY

The present disclosure provides an intermediate having a structure shown in formula (1) as follows: 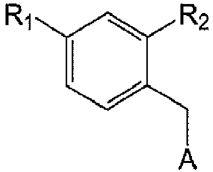 formula (1), in which R₁ is -Cl, -Br, -I, a trifluorosulfonate group (-OSO₂CF₃), -B(OH)₂, or a pinacol boronic ester group 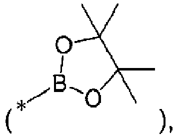 R₂ is -F, -¹⁸F, -Cl, -Br, -I, -SnMe₃, -SnBu₃, -B(OH)₂, or a pinacol boronic ester group 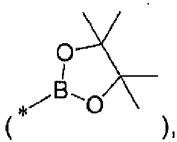 and A is a chiral auxiliary.

In some embodiments, A is 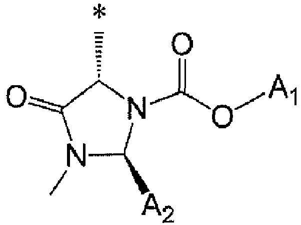 or 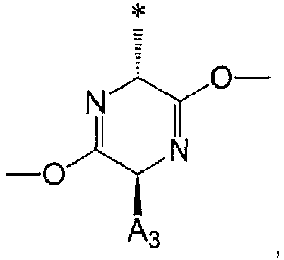 in which A₁ is a C1-C8 alkyl group, a C7-C10 aralkyl group, or a phenyl group, A₂ is a C1-C8 alkyl group, and A₃ is a C1-C8 alkyl group.

In some embodiments, A is an imidazolidinone chiral auxiliary or a bis-lactim ether chiral auxiliary.

In some embodiments, the intermediate has a structure shown in formula (2), formula (3), formula (4), formula (5), formula (6), formula (7), or formula (8), as follows: 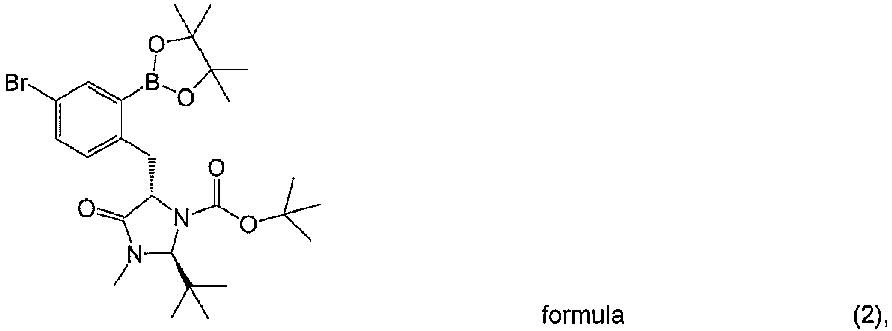 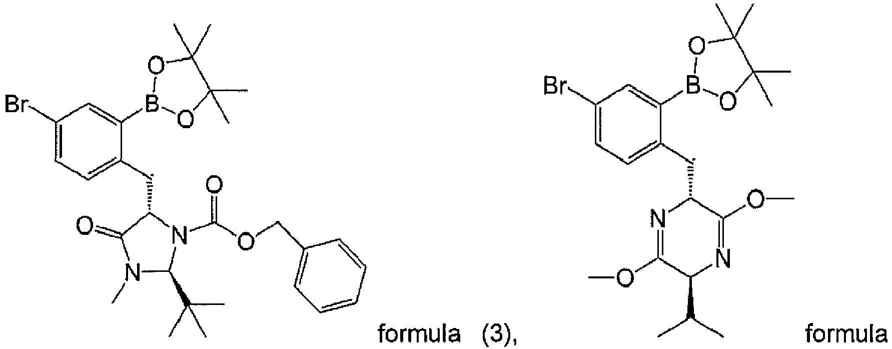 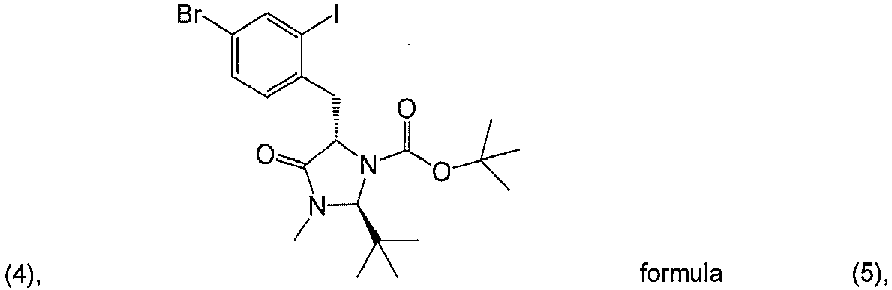 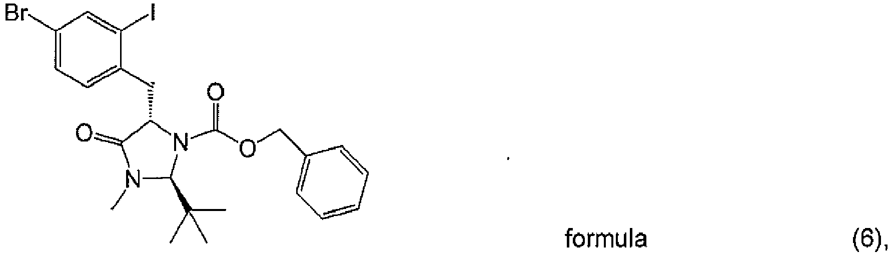 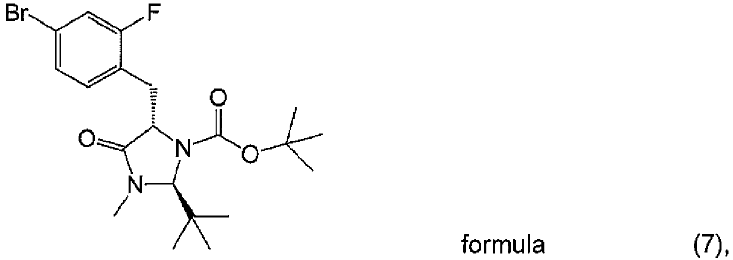 or

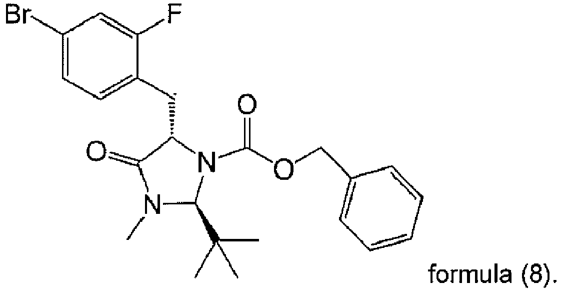

The present disclosure provides a method of preparing an intermediate including the following operations. A first reactant is reacted with a second reactant under an alkaline environment to obtain a first intermediate, in which a reaction temperature is from -80 °C to 0 °C. The first reactant has a structure shown in formula (9-1) as follows: 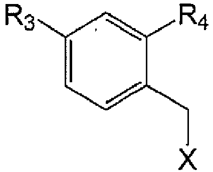 formula (9-1), R₃ is -Cl, -Br, -I, or -OSO₂CF₃₋, R₄ is -F, -Cl, -Br, -I, -SnMe₃, -SnBu₃, or -B(OH)₂, and X is -Br or -I. The second reactant has a structure shown in formula (9-2) or formula (9-3), as follows: 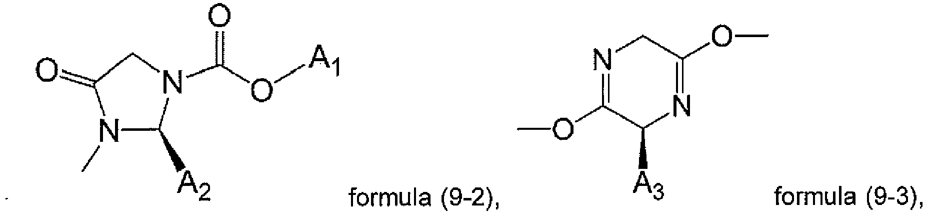 in which A₁ is a C1-C8 alkyl group, a C7-C10 aralkyl group, or a phenyl group, A₂ is a C1-C8 alkyl group, and A₃ is a C1-C8 alkyl group.

In some embodiments, the second reactant has a structure shown in formula (10), formula (11), or formula (12), as follows: 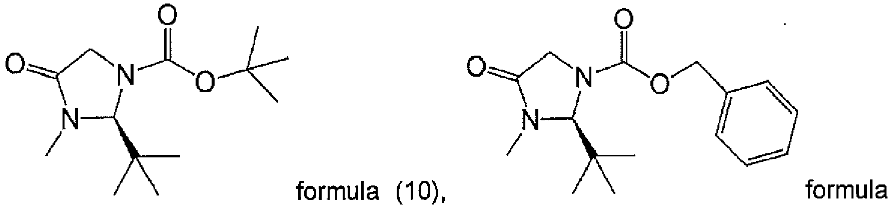 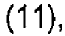 or

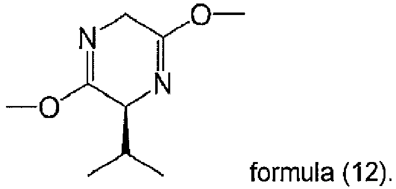

In some embodiments, reacting the first reactant with the second reactant under the alkaline environment includes: mixing the first reactant, the second reactant, and an organometallic base, the organometallic base is selected from the group consisting of lithium diisopropylamide (LDA), n-butyllithium (n-BuLi), lithium bis(trimethylsilyl)amide (LiHMDS), lithium 2,2,6,6-tetramethylpiperidine, sodium methoxide, lithium tert-butoxide, sodium tert-butoxide, potassium tert-butoxide, and sodium ethoxide.

In some embodiments, the first reactant has a structure shown in formula (13) or formula (14), as follows: 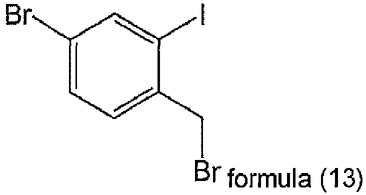 or

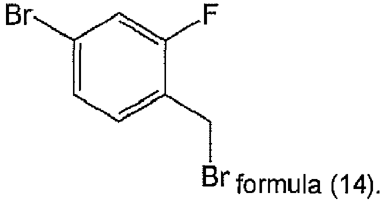

In some embodiments, the method further includes mixing the first reactant, the second reactant, and an aprotic solvent.

In some embodiments, the aprotic solvent is selected from the group consisting of tetrahydrofuran (THF), 2-methyltetrahydrofuran (2-MeTHF), dimethylformamide (DMF), dichloromethane (DCM), and dioxane.

In some embodiments, the method further includes: reacting the first intermediate with bis(pinacolato)diboron ((Bpin)₂) in the presence of a palladium catalyst to obtain a second intermediate.

In some embodiments, the method further includes mixing the first intermediate, the bis(pinacolato)diboron, and an aprotic solvent.

The present disclosure provides a method of preparing a drug including the following operations. A fluorination reaction is performed for the aforementioned intermediate and a fluorinating reagent to generate a first compound, in which in the intermediate, R₁ is -Cl, -Br, -I, or -OSO₂CF₃, and R₂ is -Cl, -Br, -I, -SnMe₃, -SnBu₃, -B(OH)₂, or

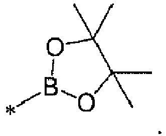

In some embodiments, performing the fluorination reaction for the intermediate and the fluorinating reagent includes: reacting the intermediate with K¹⁸F in the presence of a copper catalyst.

In some embodiments, the method further includes: performing a boronation reaction for the first compound and a boronating reagent to generate a second compound.

In some embodiments, performing the boronation reaction for the first compound and the boronating reagent includes: reacting the first compound with bis(pinacolato)diboron in the presence of a palladium catalyst.

In some embodiments, the method further includes: hydrolyzing the second compound.

### DETAILED DESCRIPTION

The present disclosure provides a preparation method of a drug. In more detail, the present disclosure provides a preparation method of ¹⁸F-labeled 2-fluoro-4-borono-phenylalanine (FBPA). The ¹⁸F-labeled FBPA is a boron-containing drug that can be applied to boron neutron capture therapy (BNCT) and positron emission tomography (PET). Since the half-life of ¹⁸F is shorter than two hours, preparing the ¹⁸F-labeled FBPA in a simple process after labeling ¹⁸F on the intermediate of the synthetic drug is very important. The present disclosure provides an intermediate and its preparation method. The ¹⁸F-labeled FBPA can be prepared from the intermediate in a simple process, thereby effectively improving the efficiency and yield of synthesizing the ¹⁸F-labeled FBPA. Also, the ¹⁸F-labeled FBPA has good specific activity. Various embodiments of the present disclosure will be described respectively in the following.

The present disclosure provides an intermediate having the structure shown in formula (1) as follows: 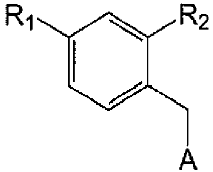 formula (1), in which R₁ is -Cl, -Br, -I, -OSO₂CF₃₋, -B(OH)₂, or 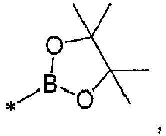 R₂ is -F, -¹⁸F, -Cl, -Br, -I, -SnMe₃, -SnBu₃, -B(OH)₂, or 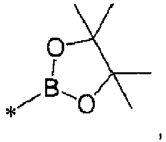 and A is a chiral auxiliary. In some embodiments, A is an imidazolidinone chiral auxiliary (also referring to Seebach's chiral auxiliary) or a bis-lactim ether chiral auxiliary (also referring to Schöllfcopf's chiral auxiliary). In some embodiments, A is 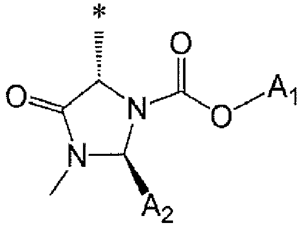 or 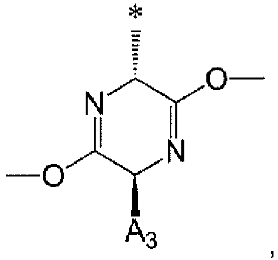 in which A₁ is a C1-C8 alkyl group, a C7-C10 aralkyl group, or a phenyl group, A₂ is a C1-C8 alkyl group, and A₃ is a C1-C8 alkyl group. The C1-C8 alkyl group is, for example, a methyl group, an ethyl group, a n-propyl group, an isopropyl group, a n-butyl group, a sec-butyl group, an isobutyl group, a tert-butyl group, a n-pentyl group, or an isopentyl group. An "aralkyl group" refers to an alkyl group having at least one hydrogen atom replaced by a phenyl group. The C7-C10 aralkyl group is, for example, a C1-C4 alkyl group having one hydrogen atom replaced by a phenyl group. In some embodiments, A is 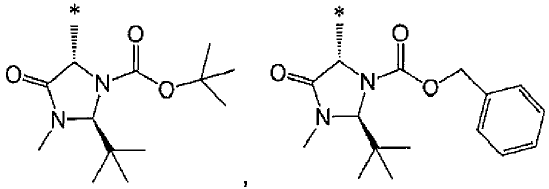 ,or 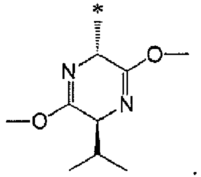 The present disclosure uses the chiral auxiliary having the specific structure to develop a chiral auxiliary precursor for synthesizing a drug, that is, an intermediate for preparing a drug. In some embodiments, the chiral auxiliary of the present disclosure can be used to develop an ¹⁸F-labeled FBPA chiral auxiliary precursor, that is, an intermediate for preparing FBPA, thereby preparing FBPA in a simple process.

In some embodiments, the intermediate has the structure shown in formula (2), formula (3), formula (4), formula (5), formula (6), formula (7), or formula (8), as follows: 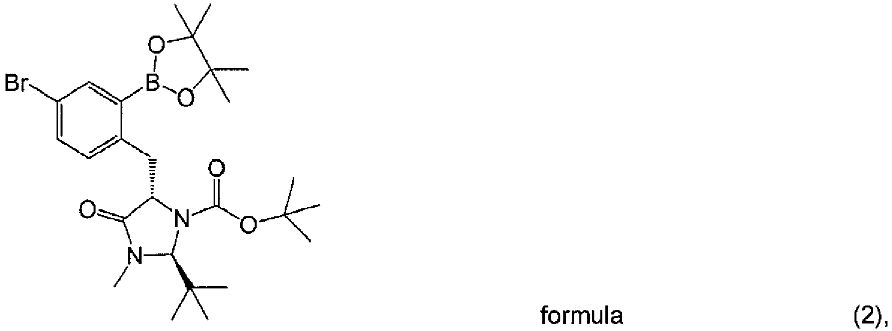 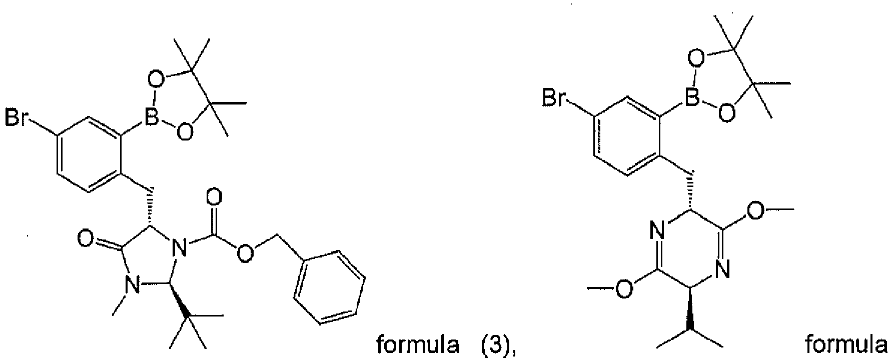 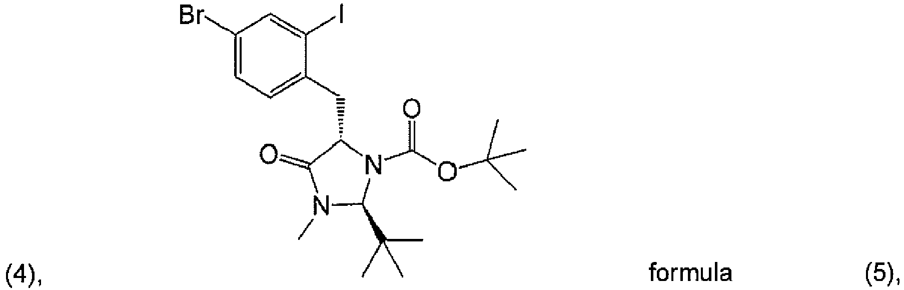 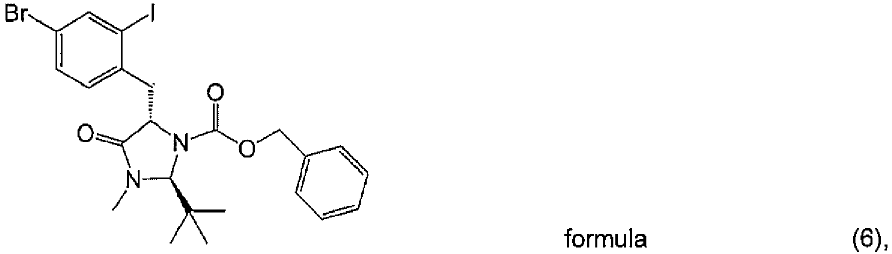 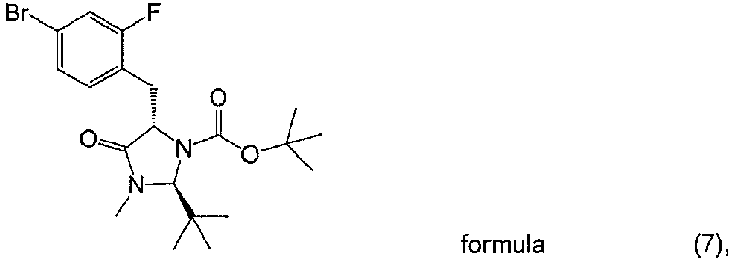 or 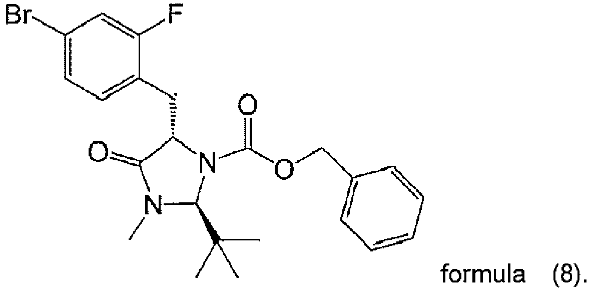 The structure having formula (2), formula (3), formula (4), formula (5), formula. (6), formula (7), or formula (8) shown above can be the intermediate in the preparation of ¹⁸F-labeled FBPA.

The present disclosure provides a method of preparing an intermediate including the following operations. A first reactant is reacted with a second reactant under an alkaline environment to obtain a first intermediate, in which a reaction temperature is from -80 °C to 0 °C. The reaction temperature is, for example, -80, -78, -76, -74, -72, -70, -60, -50, -40, -30, -20, -10, or 0 °C. The first reactant has the structure shown in formula (9-1) as follows: 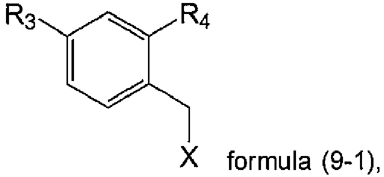 R₃ is -Cl, -Br, -I, or -OSO₂CF₃₋, R₄ is -F, -Cl, -Br, -I, -SnMe₃, -SnBu₃, or -B(OH)₂, and X is -Br or -I. The second reactant has the structure shown in formula (9-2) or formula (9-3), as follows: 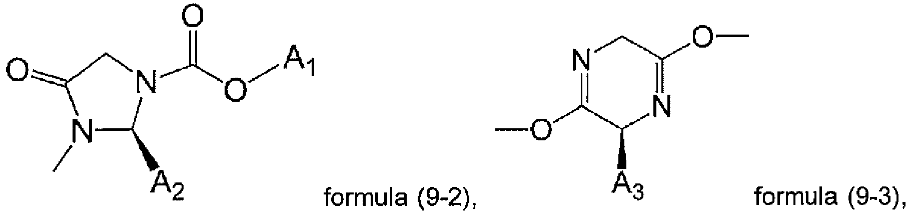 in which A₁ is a C1-C8 alkyl group, a C7-C10 aralkyl group, or a phenyl group, A₂ is a C1-C8 alkyl group, and A₃ is a C1-C8 alkyl group. Therefore, the first intermediate has the structure shown in formula (9-4) or formula (9-5), as follows: 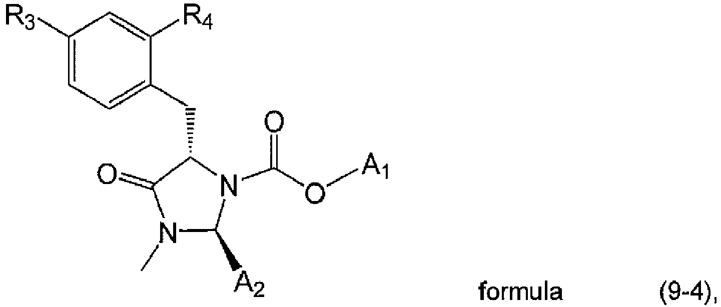 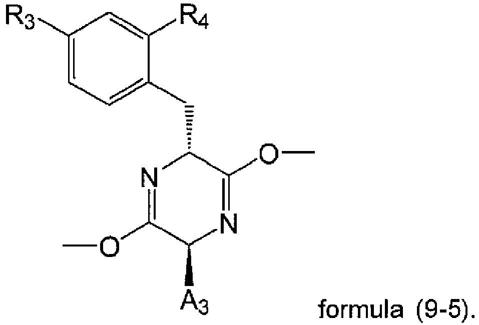 In some embodiments, the second reactant has the structure shown in formula (10), formula (11), or formula (12), as follows: 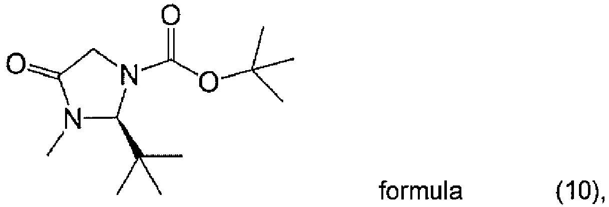 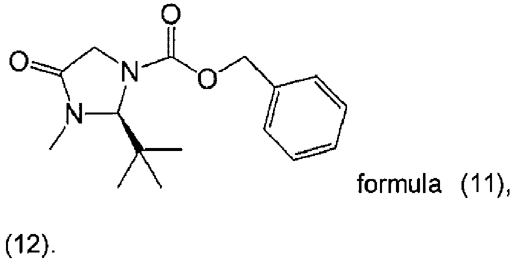 or

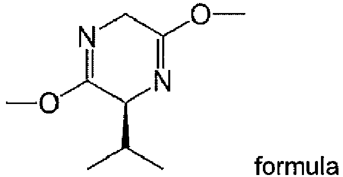

In some embodiments, the first reactant has the structure shown in formula (13) or formula (14), as follows: 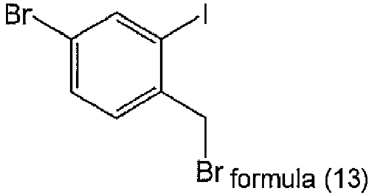 or

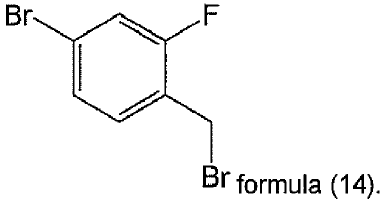

In some embodiments, reacting the first reactant with the second reactant under the alkaline environment includes: mixing the first reactant, the second reactant, and an organometallic base. The organometallic base is selected from the group consisting of lithium diisopropylamide (LDA), n-butyllithium (n-BuLi), lithium bis(trimethylsilyl)amide (LiHMDS), lithium 2,2,6,6-tetramethylpiperidine, sodium methoxide, lithium tert-butoxide, sodium tert-butoxide, potassium tert-butoxide, and sodium ethoxide.

In some embodiments, the method further includes mixing the first reactant, the second reactant, and an aprotic solvent. In some embodiments, the aprotic solvent is selected from the group consisting of tetrahydrofuran (THF), 2-methyltetrahydrofuran (2-MeTHF), dimethylformamide (DMF), dichloromethane (DCM), and dioxane.

In some embodiments, the method further includes: reacting the first intermediate with bis(pinacolato)diboron in the presence of a palladium catalyst to obtain a second intermediate. In more detail, R₄ in the first intermediate is replaced by a pinacol boronic ester group to form the second intermediate. The palladium catalyst is, for example, [1,1'-bis(diphenylphosphino)ferrocene] palladium dichloride (Pd(dppf)Cl₂). In some embodiments, the method of preparing the intermediate further includes mixing the first intermediate, the bis(pinacolato)diboron, and an aprotic solvent. The aprotic solvent please refers to the aforementioned embodiments, and the detail is not repeated herein. In some embodiments, a temperature for reacting the first intermediate with the bis(pinacolato)diboron is from 50 °C to 120 °C, for example, 50, 55, 60, 65, 70, 75, 80, 85, 90, 95, 100, 105, 110, 115, or 120 °C.

The present disclosure provides a method of preparing a drug including: performing a fluorination reaction for the intermediate and a fluorinating reagent to generate a first compound, in which in the intermediate, R₁ is -Cl, -Br, -I, or -OSO₂CF₃, and R₂ is -Cl, -Br, -I, -SnMe₃, -SnBu₃, -B(OH)₂, or 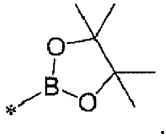 In some embodiments, the fluorinating agent is a metal fluoride, for example, K¹⁸F. In some embodiments, performing the fluorination reaction for the intermediate and the fluorinating reagent includes: reacting the intermediate with K¹⁸F in the presence of a copper catalyst. The copper catalyst is, for example, Cu(OTf)₂(py)₄. In some embodiments, the method further includes performing a boronation reaction for the first compound and a boronating reagent to generate a second compound. The boronating reagent is, for example, bis(pinacolato)diboron ((Bpin)₂). In some embodiments, performing the boronation reaction for the first compound and the boronating reagent includes: reacting the first compound with bis(pinacolato)diboron in the presence of a palladium catalyst. In some embodiments, the method further includes hydrolyzing the second compound.

The present disclosure provides a method of preparing ¹⁸F-labeled 2-fluoro-4-borono-phenylalanine (FBPA), including the following operations. A fluorination reaction is performed for the intermediate to generate a first compound, in which the intermediate has the structure shown in formula (1) as follows: 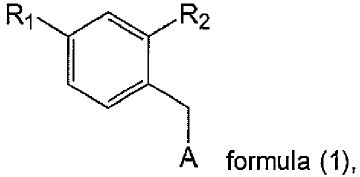 R₁ is -Cl, -Br, -I, or -OSO₂CF₃, R₂ is -Cl, -Br, -I, -SnMe₃, -SnBu₃, -B(OH)₂, or 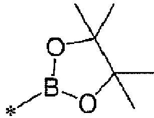 , and the first compound has the structure shown in formula (X) as follows: 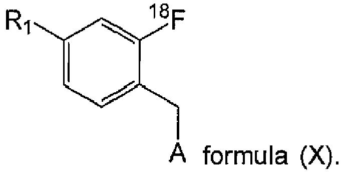 A boronation reaction is performed for the first compound to generate a second compound. The second compound has the structure shown in formula (XI) as follows: 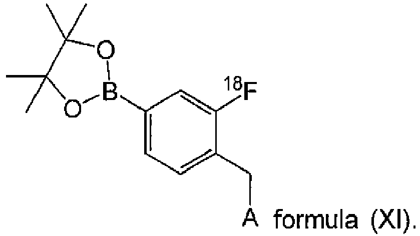 The second compound is hydrolyzed to generate 2-fluoro-4-borono-phenylalanine having the structure shown in formula (XII) as follows:

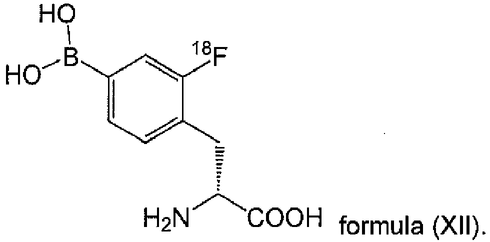

In some embodiments, performing the fluorination reaction for the intermediate includes: reacting the intermediate with K¹⁸F in the presence of a copper catalyst, so that the R₂ on the aromatic ring is replaced by ¹⁸F. In other words, the aromatic ring of the intermediate is fluorinated by ¹⁸F. The above-mentioned fluorination reaction catalyzed by the copper catalyst is called copper-mediated aromatic ring fluorination reaction. The copper catalyst is, for example, Cu(OTf)₂(py)₄. In some embodiments, a temperature for the fluorination reaction is from 100 °C to 120 °C. The temperature is, for example, 100, 105, 110, 115, or 120 °C. In some embodiments, a reaction time of the fluorination reaction is from 5 minutes to 60 minutes. K¹⁸F has the best reactivity with the pinacol boronic ester group 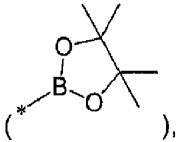 followed by the other groups, such as -Cl, -Br, -I, -SnMe₃, -SnBu₃, or -B(OH)₂, etc. In some embodiments, K¹⁸F can be obtained from the following operations: irradiating H₂¹⁸O with an accelerated proton, using ¹⁸O(p, n)¹⁸F to react and synthesize hydrofluoric acid (H¹⁸F), and then passing the H¹⁸F through an ion exchange column to adsorb it on the column and separate it from the H₂¹⁸O not adsorbed on the column. The H¹⁸F in the column is eluted with K₂CO₃ aqueous solution to obtain K¹⁸F. In some embodiments, when performing the fluorination reaction, the intermediate, K¹⁸F and the copper catalyst are dissolved in an aprotic solvent, for example, dimethylformamide (DMF). The material of the aprotic solvent please refers to the foregoing embodiments, and the detail is not repeated herein.

In some embodiments, performing the boronation reaction for the first compound includes: reacting the first compound with bis(pinacolato)diboron ((Bpin)₂) in the presence of a palladium catalyst, thereby making the R₁ on the aromatic ring replaced by a pinacol boronic ester group. The above-mentioned boronation reaction catalyzed by the palladium catalyst is called Miyaura boronation reaction. The bis(pinacolato)diboron has the best reactivity with -Br, followed by the other groups, such as -Cl, -I, or -OSO₂CF₃, etc. The palladium catalyst is, for example, tris(dibenzylideneacetone)dipalladium (Pd₂(dba)₃), which uses tricyclohexylphosphine (P(Cy)₃) as a ligand. In some embodiments, Pd₂(dba)₃ can be dissolved in dioxane. The palladium catalyst is, for example, [1,1'-bis(diphenylphosphino)ferrocene]palladium dichloride (Pd(dppf)Cl₂). In some embodiments, PdCl₂(dppf) can be dissolved in potassium acetate (KOAc). In some embodiments, a temperature for the boronation reaction is from 70 °C to 120 °C. The temperature is, for example, 70, 75, 80, 85, 90, 95, 100, 105, 110, 115, or 120 °C. In some embodiments, a reaction time for the boronation reaction is from 5 minutes to 60 minutes. In some embodiments, when performing the boronation reaction, the first compound and the bis(pinacolato)diboron are dissolved in an aprotic solvent, for example, dimethylsulfoxide (DMSO) or dioxane. The material of the aprotic solvent please refers to the foregoing embodiments, and the detail is not repeated herein.

In some embodiments, hydrolyzing the second compound includes: reacting the second compound with an acid. The acid is, for example, hydrogen chloride (HCl), hydrogen bromide (HBr), hydrogen iodide (HI), or combinations thereof. The acid can open the pinacol boronic ester group 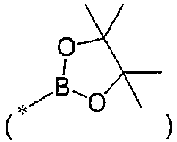 of the second compound and hydrolyzes it into -B(OH)₂. Also, the acid can hydrolyze the chiral auxiliary A of the second compound into an amino acid group. In some embodiments, a temperature for the hydrolysis is from 130 °C to 170 °C. The temperature is, for example, 130, 140, 150, 160, or 170 °C. In some embodiments, a reaction time for the hydrolysis is from 5 minutes to 60 minutes.

### Experimental example 1: preparing the intermediates having formula (5) and formula (2)

Firstly, the intermediate having formula (5) is prepared. Please refer to the following reaction formula for the preparation process.

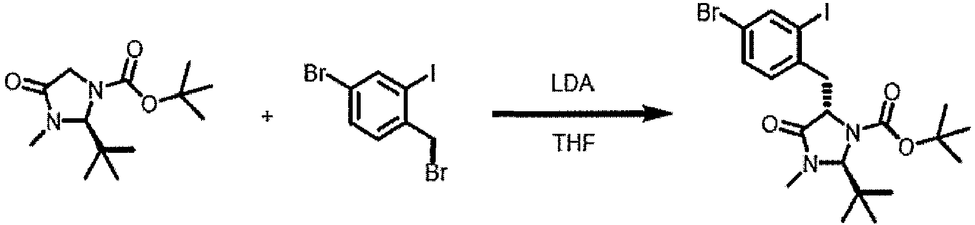

The preparation process is described in detail below. The compound of formula (10) (3.0 g) and THF (40 ml), i.e., (S)-tert-butyl 2-tert-butyl-3-methyl-4-oxoimidazolidine-1-carboxylate and THF, were added into a 100 ml reaction flask. °C The solution was stirred until the solute was completely dissolved. The solution temperature was lowered to -75 °C to -70 °C in nitrogen. LDA solution (14.0 ml) was added dropwise to the solution, and the temperature was controlled at -75 °C to -70 °C. In the LDA solution, the concentration of LDA was 1M, and LDA was dissolved in THF/hexane. The solution temperature was kept at -75 °C to -70 °C, and the mixture solution was stirred for 30 minutes. A mixture solution of 4-bromo-1-(bromomethyl)-2-iodobenzene (4.8 g) and THF (5 ml) was added dropwise to the solution. The solution temperature was controlled at -75 °C to -70 °C. The solution was warmed to room temperature (room temperature herein is about 25° C), and stirred for 16 hours. The solution was extracted with saturated ammonium chloride aqueous solution (50 ml) and dichloromethane (60 ml) to obtain a first organic layer and an aqueous layer. The aqueous layer was extracted twice with dichloromethane (30 ml) to obtain a second organic layer. The first organic layer and the second organic layer were combined as an organic layer extract. The organic layer extract was dried with anhydrous sodium sulfate, filtered, and then concentrated to obtain a concentrated solution. The concentrated solution was purified by flash column chromatography (ethyl acetate (EA): heptane=1:5) to obtain the product (4.2 g, 65%). The chemical shifts (δ) (unit: ppm) of the proton nuclear magnetic resonance (NMR) (¹H-NMR (400 MHz, CDCl₃)) of the product are 0.99 (s, 9H), 1.31 (s, 9H), 3.01 (s, 3H), 3.38 (dd, 1H, J=16.4, 6.8 Hz), 3.52 (dd, 1H, J=16.4, 3.2 Hz), 4.35 (dd, 1H, J=6.0, 4.0 Hz), 5.06 (s, 1H), 6.78 (d, 1H, J=8.4 Hz), 7.35 (dd, 1H, J=8.4, 2.0 Hz), 7.97 (d, 1H, J=1.6 Hz). Proton NMR can prove that experimental example 1 can prepare the aforementioned intermediate having formula (5) in the present disclosure, and its structure name is (2S,5S)-tert-butyl 5-(4-bromo-2-iodobenzyl)-2-tert-butyl-3-methyl-4-oxoi midazolidi ne-1-carboxylate.

Next, the intermediate having formula (2) is prepared from the intermediate having formula (5). Please refer to the following reaction formula for the preparation process.

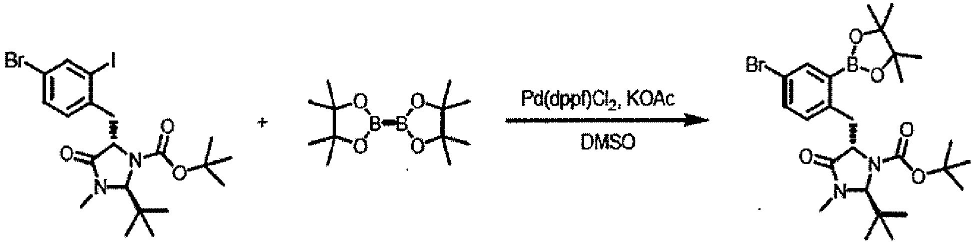

The preparation process is described in detail below. DMSO (5 ml) was added to a 25 ml reaction flask and bubbled with argon for 30 minutes. The intermediate having formula (5) (580 mg), bis(pinacolato)diboron (588 mg), potassium acetate (KOAc) (313 mg), and the palladium catalyst Pd(dppf)Cl₂ (38.5 mg) were added and continually bubbled with argon for 10 minutes. The solution was heated under argon, and the temperature of the solution was controlled to be 80° C to allow the solution to react for 24 hours. The temperature of the solution was lowered to room temperature. The solution was extracted with water (15 ml) and dichloromethane (20 ml) to obtain a first organic layer and an aqueous layer, and the aqueous layer was extracted twice with dichloromethane (15 ml) to obtain a second organic layer. The first organic layer and the second organic layer were combined into an organic layer extract, and the organic layer extract was extracted with water (15 ml). The organic layer extract was dried with anhydrous sodium sulfate, filtered, and concentrated to obtain a concentrated solution. The concentrated solution was purified by flash column chromatography (EA: heptane=1:9) to obtain the product (211 mg, 36%). The chemical shifts (δ) (unit: ppm) of the proton NMR (¹H-NMR (400 MHz, CDCl₃)) of the product are 0.93 (s, 9H), 1.36 (s, 6H), 1.37 (s, 6H), 1.40 (s, 9H), 2.77 (s, 3H), 3.69 (dd, 1H, J=15.0, 2.6 Hz), 3.83 (br, 1H), 4.29 (t, 1H, J=3.6 Hz), 4.77 (s, 1H), 6.82 (d, 1H, J=8.4 Hz), 7.37 (dd, 1H, J=8.4, 2.4 Hz), 7.82 (d, 1H, J=2.4 Hz). Proton NMR can prove that experimental example 1 can prepare the aforementioned intermediate having formula (2) in the present disclosure, and its structure name is (2S,5S)-tert-butyl 5-(4-bromo-2-(4,4,5,5-tetramethyl-1,3,2-dioxaborolan-2-yl)benzyl)-2-tert-buty[-3-methyl-4-oxoimidazolidine-1-carboxylate.

### Experimental example 2: preparing the intermediates having formula (6) and formula (3)

Firstly, the intermediate having formula (6) is prepared. Please refer to the following reaction formula for the preparation process.

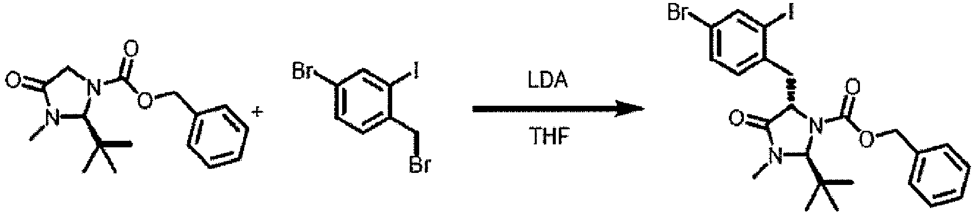

The preparation process is described in detail below. The compound having formula (11) (3.9 g) and THF (40 ml), i.e., (S)-benzyl 2-tert-butyl-3-methyl-4-oxoimidazolidine-1-carboxylate and THF, were added into a 100 ml reaction flask. The solution was stirred until the solute was completely dissolved. The solution temperature was lowered to -75 °C to -70 °C in nitrogen. LDA solution (16.2 ml) was added dropwise to the solution, and the temperature was controlled at -75 °C to -70 °C. In the LDA solution, the concentration of LDA was 1M, and LDA was dissolved in THF/hexane. The solution temperature was kept at -75 °C to -70 °C and the mixture solution was stirred for 30 min. A mixture solution of 4-bromo-1-(bromomethyl)-2-iodobenzene (5.6 g) and THF (5 ml) was added dropwise to the solution, and the temperature of the solution was controlled at -75 °C to -70 °C. The solution was warmed to room temperature and stirred for 16 hours. The solution was extracted with saturated ammonium chloride aqueous solution (50 ml) and dichloromethane (60 ml) to obtain a first organic layer and an aqueous layer, and the aqueous layer was extracted twice with dichloromethane (30 ml) to obtain a second organic layer. The first organic layer and the second organic layer were combined into an organic layer extract, which was dried with anhydrous sodium sulfate, filtered, and then concentrated to obtain a concentrated solution. The concentrated solution was purified by flash column chromatography (EA: heptane=1:5) to obtain the product (5.5 g, 70%). The chemical shifts (δ) (unit: ppm) of the proton NMR (¹H-NMR (400 MHz, CDCl₃)) of the product are 0.98 (s, 9H), 3.00 (s, 3H), 3.32 (dd, 1H, J=15.6, 6.4 Hz), 3.53 (d, 1H, J=15.2 Hz), 4.40 (t, 1H, J=5.2 Hz), 4.89 (d, 1H, J=12.0 Hz), 5.09 (s, 1H), 5.15 (d, 1H, J=12.0 Hz), 6.82 (d, 1H, J=8.4 Hz), 7.21-7.32 (m, 6H), 7.90 (s, 1H). Proton NMR can prove that experimental example 2 can prepare the aforementioned intermediate having formula (6) in the present disclosure, and its structure name is (2S,5S)-benzyl 5-(4-bromo-2-iodobenzyl)-2-tert-butyl-3-methyl-4-oxoimidazolidine-1-carboxylate.

Next, the intermediate having formula (3) is prepared from the intermediate having formula (6). Please refer to the following reaction formula for the preparation process.

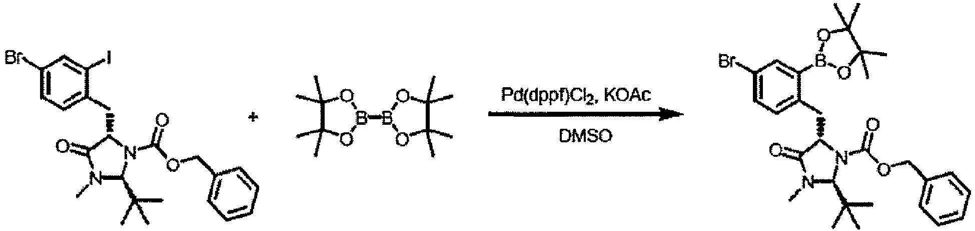

The preparation process is described in detail below. DMSO (5 ml) was added to a 25 ml reaction flask and bubbled with argon for 30 minutes. The intermediate having formula (6) (500 mg), bis(pinacolato)diboron (477 mg), potassium acetate (254 mg), and the palladium catalyst Pd(dppf)Cl₂ (31.3 mg) were added and continually bubbled with argon for 10 minutes. The solution was heated under argon, and the temperature of the solution was controlled to be 80° C to allow the solution to react for 22 hours. The temperature of the solution was lowered to room temperature. The solution was extracted with water (15 ml) and dichloromethane (20 ml) to obtain a first organic layer and an aqueous layer, and the aqueous layer was extracted twice with dichloromethane (15 ml) to obtain a second organic layer. The first organic layer and the second organic layer were combined into an organic layer extract, and the organic layer extract was extracted with water (15 ml). The organic layer extract was dried with anhydrous sodium sulfate, filtered, and concentrated to obtain a concentrated solution. The concentrated solution was purified by flash column chromatography (EA: heptane=1:7) to obtain the product (246 mg, 49%). The chemical shifts (δ) (unit: ppm) of the proton NMR (¹H-NMR (400 MHz, CDCl₃)) of the product are 0.88 (s, 9H), 1.37 (s, 12H), 2.78 (s, 3H), 3.64 (d, 1H, J=12.8 Hz), 3.88 (br, 1H), 4.35 (t, 1H, J=3.2 Hz), 4.69 (s, 1H), 4.93 (d, 1H, J=11.6 Hz), 5.20 (d, 1H, J=12.0 Hz), 6.72 (d, 1H, J=8.0 Hz), 7.25-7.36 (m, 6H), 7.79 (d, 1H, J=2.4 Hz). Proton NMR can prove that experimental example 2 can prepare the aforementioned intermediate having formula (3) in the present disclosure, and its structure name is (2S,5S)-benzyl 5-(4-bromo-2-(4,4,5,5-tetramethyl-1,3,2-dioxaborolan-2-yl)benzyl)-2-tert-butyl-3-methyl-4- oxoimidazolidine-1-carboxylate.

### Experimental example 3: preparing the intermediate having formula (7)

Please refer to the following reaction formula for the preparation process of the intermediate having formula (7).

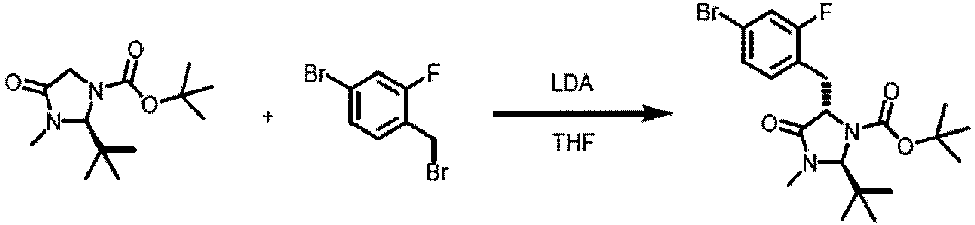

The preparation process is described in detail below. The compound having formula (10) (200 mg) and THF (5 ml), i.e., (S)-benzyl 2-tert-butyl-3-methyl-4-oxoimidazolidine-1-carboxylate and THF, were added into a 100 ml reaction flask. The solution was stirred until the solute was completely dissolved. The solution temperature was lowered to -75 °C to -70 °C in nitrogen. LDA solution (0.94 ml) was added dropwise to the solution, and the temperature was controlled at -75 °C to -70 °C. In the LDA solution, the concentration of LDA was 1M, and LDA was dissolved in THF/hexane. The solution temperature was kept at -75 °C to -70 °C and the mixture solution was stirred for 30 min. A mixture solution of 4-bromo-1-(bromomethyl)-2-fluorobenzene (209 mg) and THF (2 ml) was added dropwise to the solution, and the solution temperature was control at -75 °C to -70 °C. The solution was warmed to room temperature and stirred for 16 hours. The solution was extracted with saturated ammonium chloride aqueous solution (10 ml) and dichloromethane (15 ml) to obtain a first organic layer and an aqueous layer, and the aqueous layer was extracted twice with dichloromethane (10 ml) to obtain a second organic layer. The first organic layer and the second organic layer were combined into an organic layer extract, which was dried with anhydrous sodium sulfate, filtered, and then concentrated to obtain a concentrated solution. The concentrated solution was purified by flash column chromatography (EA: heptane=1:5) to obtain the product (105 mg, 30%). The chemical shifts (δ) (unit: ppm) of the proton NMR (¹H-NMR (400 MHz, CDCl₃)) of the product are 0.95 (s, 9H), 1.41 (s, 9H), 2.87 (s, 3H), 3.31 (dd, 1H, J=15.0, 2.2 Hz), 3.54 (br, 1H), 4.31 (s, 1H), 4.83 (s, 1 H), 6.96 (t, 1H, J=8.2 Hz), 7.13-7.17 ( m, 2H). Proton NMR can prove that experimental example 3 can prepare the aforementioned intermediate having formula (7) in the present disclosure, and its structure name is (2S,5S)-tert-butyl 5-(4-bromo-2-fluorobenzyl)-2-tert-butyl-3-methyl-4-oxoimidazolidine-1-carboxyla te. The intermediate having formula (7) can further react with bis(pinacolato)diboron to replace the F group with a pinacol boronic ester group. The product can be used to prepare FBPA. The reaction process can refer to experimental example 1 or experimental example 2.

### Experimental example 4: preparing the intermediate having formula (8)

Please refer to the following reaction formula for the preparation process of the intermediate having formula (8). 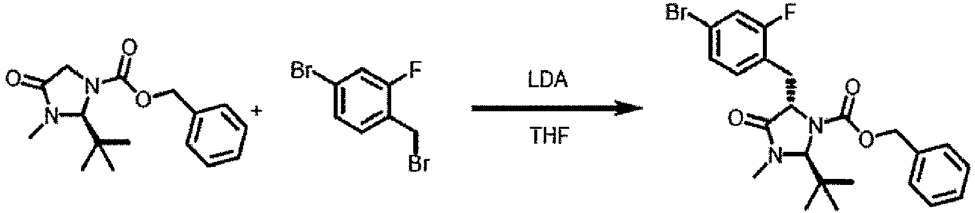 The preparation process is described in detail below. The compound having formula (11) (200 mg) and THF (5 ml), i.e., (S)-benzyl 2-tert-butyl-3-methyl-4-oxoimidazolidine-1-carboxylate and THF, were added into a 100 ml reaction flask. The solution was stirred until the solute was completely dissolved. The solution temperature was lowered to -75 °C to -70 °C in nitrogen. LDA solution (0.83 ml) was added dropwise to the solution, and the temperature was controlled at -75 °C to -70 °C. In the LDA solution, the concentration of LDA was 1M, and LDA was dissolved in THF/hexane. The solution temperature was kept at -75 °C to -70 °C and the mixture solution was stirred for 30 minutes. A mixture solution of 4-bromo-1-(bromomethyl)-2-fluorobenzene (185 mg) and THF (2 ml) was added dropwise to the solution, and the solution temperature was controlled at -75 °C to -70 °C. The solution was warmed to room temperature and stirred for 16 hours. The solution was extracted with saturated ammonium chloride aqueous solution (10 ml) and dichloromethane (15 ml) to obtain a first organic layer and an aqueous layer, and the aqueous layer was extracted twice with dichloromethane (10 ml) to obtain a second organic layer. The first organic layer and the second organic layer were combined into an organic layer extract, which was dried with anhydrous sodium sulfate, filtered, and then concentrated to obtain a concentrated solution. The concentrated solution was purified by flash column chromatography (EA: heptane=1:4) to obtain the product (77 mg, 23%). The chemical shifts (δ) (unit: ppm) of the proton NMR (¹H-NMR (400 MHz, CDCl₃)) of the product are 0.91 (s, 9H), 2.84 (s, 3H), 3.19 (d, 1H, J=14.0 Hz), 3.65 (dd, 1H, J=14.0, 3.2 Hz), 4.38 (s, 1H), 4.81 (s, 1H), 4.96 (d, 1H, J=12.0 Hz), 5.26 (d, 1H, J=12.0 Hz), 6.96 (t, 1H, J=8.0 Hz), 7.08-7.14 (m, 2H), 7.35-7.39 (m, 5H). Proton NMR can prove that experimental example 4 can prepare the aforementioned intermediate having formula (8) in the present disclosure, and its structure name is (2S,5S)-benzyl 5-(4-bromo-2-fluorobenzyl)-2-tert-butyl-3-methyl-4-oxoimidazolidine-1-carboxyla te. The intermediate having formula (8) can further react with bis(pinacolato)diboron to replace the F group with a pinacol boronic ester group. The product can be used to prepare FBPA. The reaction process can refer to experimental example 1 or experimental example 2.

### Experimental example 5: preparing ¹⁸F-labeled FBPA with the intermediate having the structure of formula (2)

Please refer to the following reaction formula for the preparation process.

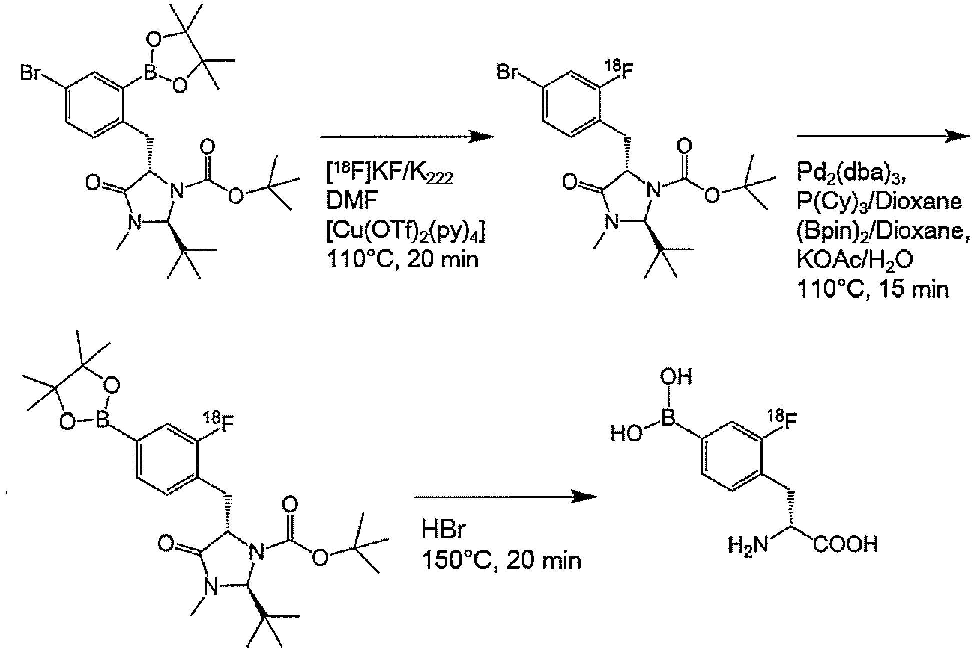

The preparation process is described in detail below. The intermediate having the structure of formula (2) was the starting reactant to perform the fluorination reaction in the presence of the copper catalyst Cu(OTf)₂(py)₄. The intermediate reacts with K¹⁸F to replace the pinacol boronic ester group on the aromatic ring by ¹⁸F. In the fluorination reaction, the solvent was dimethylformamide (DMF), K₂₂₂ is amino polyether, the reaction temperature was 110 °C, and the reaction time was 20 minutes. Next, the boronation reaction was performed, in which the fluorinated intermediate reacts with bis(pinacolato)diboron ((Bpin)₂) in the presence of the palladium catalyst Pd₂(dba)₃ to replace the -Br on the aromatic ring by the pinacol boronic ester group. P(Cy)₃ is the ligand of the palladium catalyst Pd₂(dba)₃. In the boronation reaction, Pd₂(dba)₃ and (Bpin)₂ can be dissolved into dioxane, the solvent can be potassium acetate (KOAc) and water, the reaction temperature was 110 °C, and the reaction time was 15 minutes. Next, the hydrolysis reaction was performed to react the intermediate with hydrogen bromide (HBr). HBr can hydrolyze the pinacol boronic ester group 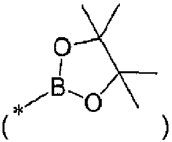 into -B(OH)₂, and the chiral auxiliary into amino acid group. In the hydrolysis reaction, the reaction temperature was 150 °C and the reaction time was 20 minutes.

### Experimental example 6: preparing ¹⁸F-labeled FBPA with the intermediate having the structure of formula (3)

Please refer to the following reaction formula for the preparation process.

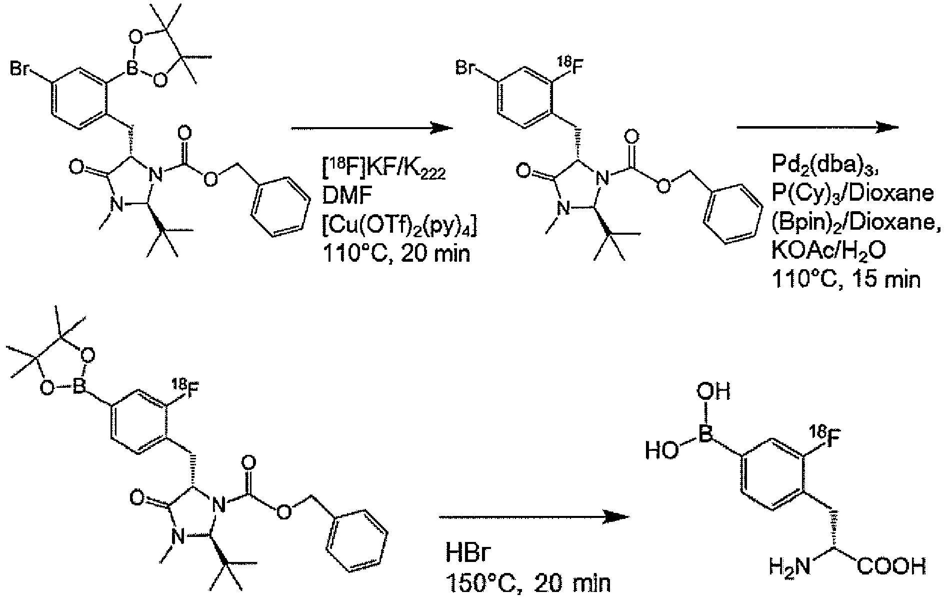

The preparation process is described in detail below. The ¹⁸F-labeled FBPA is prepared from the intermediate having the structure of formula (3) being the starting reactant. Please refer to the above-mentioned experimental example 5 for the reaction conditions of the experimental example 6. The difference between experimental example 5 and experimental example 6 is the different starting reactants, so the reaction process of experimental example 6 is not repeated herein.

In summary, the present disclosure provides a method of preparing a drug, an intermediate for synthesizing the drug, and a method of preparing the intermediate, especially relating to the preparation method of ¹⁸F labeled FBPA, the intermediate used in the synthesis of FBPA, and the preparation method of the intermediate. The process of the preparation method of FBPA in the present disclosure is simple for effectively improving the efficiency and yield of synthesizing FBPA. Also, FBPA can have good specific activity.

## Claims

1. An intermediate, having a structure shown in formula (1) as follows: 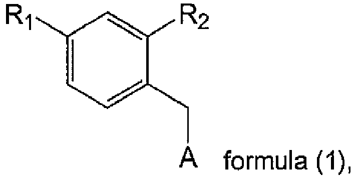 wherein R₁ is -Cl, -Br, -I, -OSO₂CF₃, -B(OH)₂, or 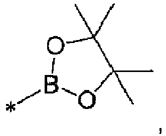 R₂ is -F, -¹⁸F, -Cl, -Br, -I, -SnMe₃, -SnBu₃, -B(OH)₂, or auxiliary. 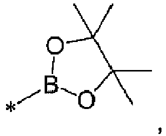 and A is a chiral

2. The intermediate as claimed in claim 1, wherein A is 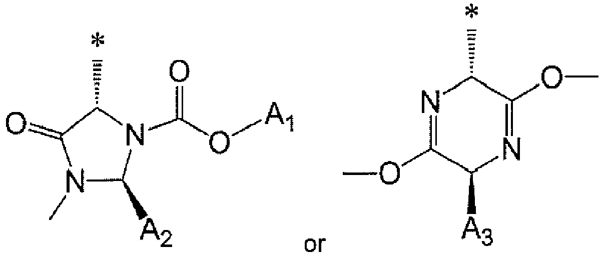 , wherein A₁ is a C1-C8 alkyl group, a C7-C10 aralkyl group, or a phenyl group, A₂ is a C1-C8 alkyl group, and A₃ is a C1-C8 alkyl group.

3. The intermediate as claimed in claim 1, wherein A is an imidazolidinone chiral auxiliary or a bis-lactim ether chiral auxiliary.

4. The intermediate as claimed in claim 2, wherein the intermediate has a structure shown in formula (2), formula (3), formula (4), formula (5), formula (6), formula (7), or formula (8), as follows: 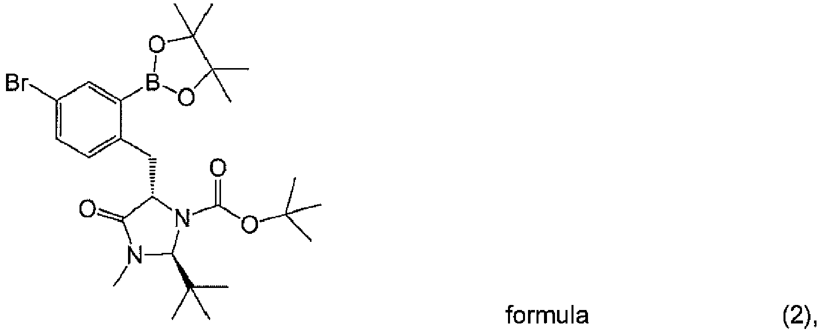 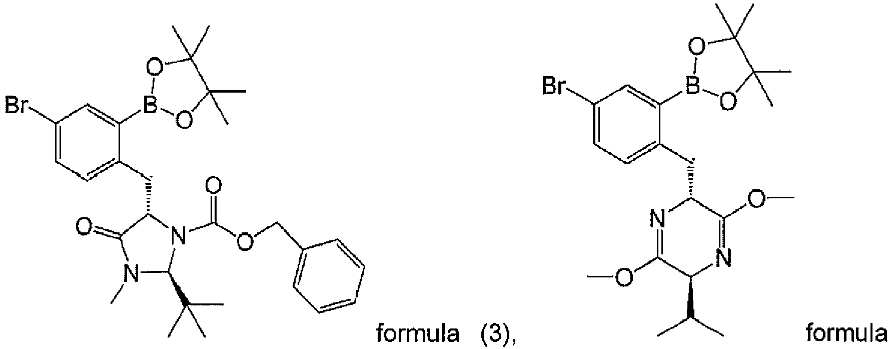 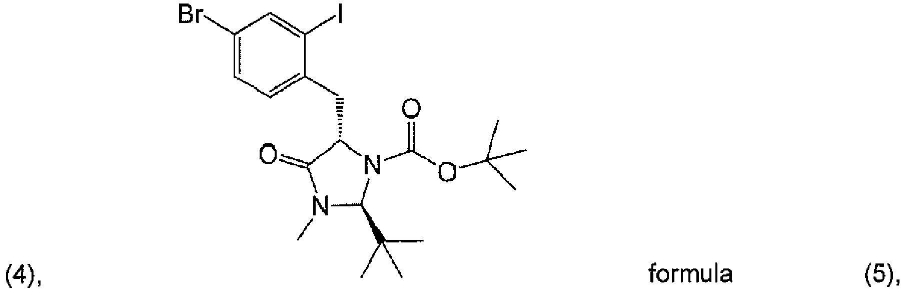 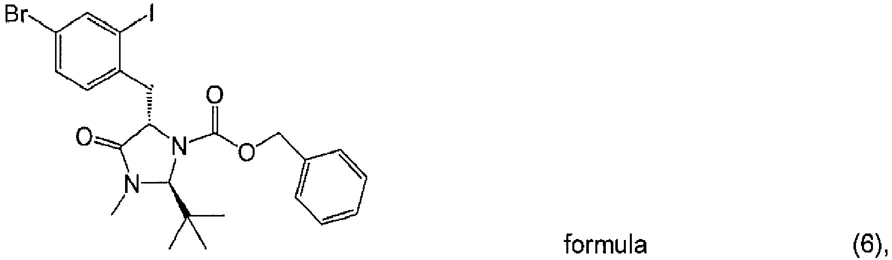 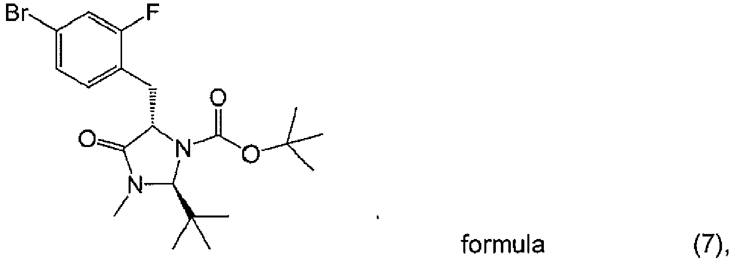 or

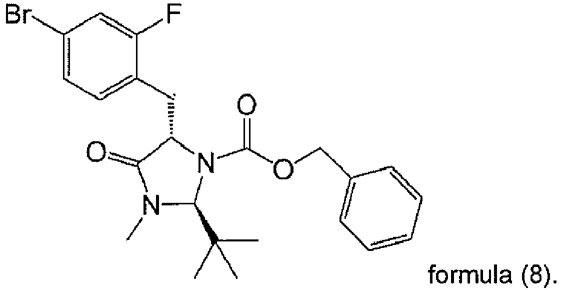

5. A method of preparing an intermediate, comprising:
reacting a first reactant with a second reactant under an alkaline environment to obtain a first intermediate, a reaction temperature being from -80 °C to 0 °C,
wherein the first reactant has a structure shown in formula (9-1) as follows: 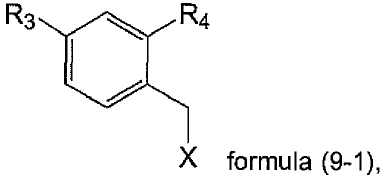 R₃ is -Cl, -Br, -I, or -OSO₂CF₃₋, R₄ is -F, -Cl, -Br, -I, -SnMe₃, -SnBu₃, or -B(OH)₂, and X is -Br or -I; and
the second reactant has a structure shown in formula (9-2) or formula (9-3), as follows: 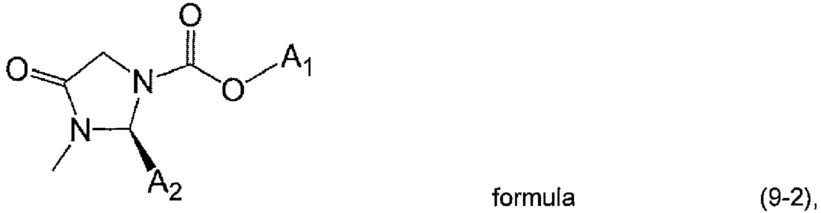 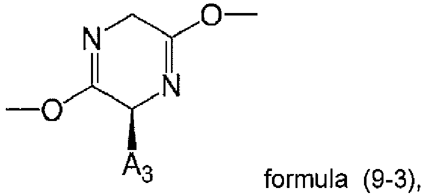 wherein A₁ is a C1-C8 alkyl group, a C7-C10 aralkyl group, or a phenyl group, A₂ is a C1-C8 alkyl group, and A₃ is a C1-C8 alkyl group.

6. The method as claimed in claim 5, wherein the second reactant has a structure shown in formula (10), formula (11), or formula (12), as follows: 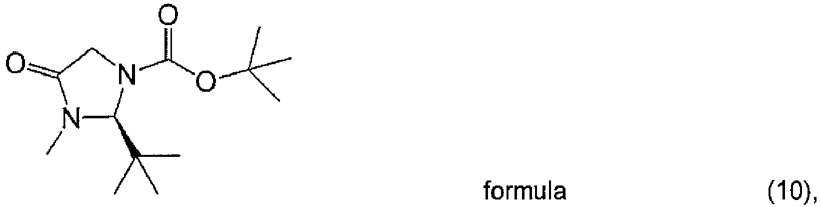 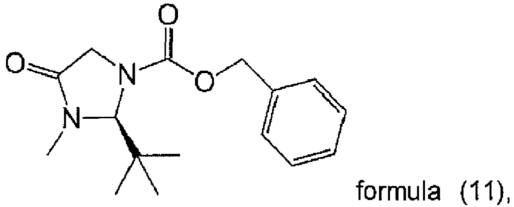 or

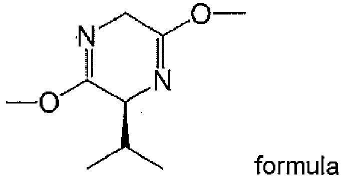

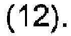

7. The method as claimed in claim 5 or claim 6, wherein reacting the first reactant with the second reactant under the alkaline environment comprises: mixing the first reactant, the second reactant, and an organometallic base, the organometallic base is selected from the group consisting of lithium diisopropylamide, n-butyllithium, lithium bis(trimethylsilyl)amide, lithium 2,2,6,6-tetramethylpiperidine, sodium methoxide, lithium tert-butoxide, sodium tert-butoxide, potassium tert-butoxide, and sodium ethoxide.

8. The method as claimed in any one of claims 5-7, wherein the first reactant has a structure shown in formula (13) or formula (14), as follows: 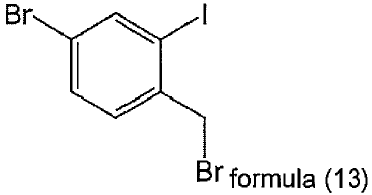 or

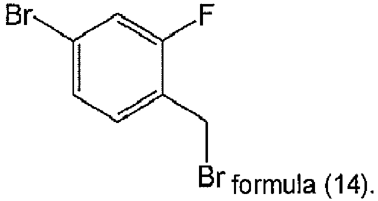

9. The method as claimed in any one of claims 5-8, further comprising:
reacting the first intermediate with bis(pinacolato)diboron in the presence of a palladium catalyst to obtain a second intermediate.

10. The method as claimed in claim 9, further comprising mixing the first intermediate, the bis(pinacolato)diboron, and an aprotic solvent.

11. A method of preparing a drug, comprising:
performing a fluorination reaction for the intermediate as claimed in claim 1 and a fluorinating reagent to generate a first compound, wherein in the intermediate, R₁ is -Cl, -Br, -I, or -OSO₂CF₃, and R₂ is -Cl, -Br, -I, -SnMe₃, -SnBu₃, -B(OH)₂, or

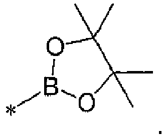

12. The method as claimed in claim 11, wherein performing the fluorination reaction for the intermediate as claimed in claim 1 and the fluorinating reagent comprises:
reacting the intermediate as claimed in claim 1 with K¹⁸F in the presence of a copper catalyst.

13. The method as claimed in claim 11 or claim 12, further comprising: performing a boronation reaction for the first compound and a boronating reagent to generate a second compound.

14. The method as claimed in claim 13, wherein performing the boronation reaction for the first compound and the boronating reagent comprises:
reacting the first compound with bis(pinacolato)diboron in the presence of a palladium catalyst.

15. The method as claimed in claim 13 or claim 14, further comprising: hydrolyzing the second compound.
